# EUROPEAN PATENT APPLICATION

(11) **EP 4 035 776 A1**
(43) Date of publication of application: **03.08.2022**
(21) Application number: 21154133.9
(22) Date of filing: 28.01.2021
(51) Int. Cl.: B01L 3/00

(54) **DEVICES AND METHODS FOR ACOUSTOFLUIDIC OPERATIONS USING THIN FILM ULTRASOUND TRANSDUCERS**

(71) Applicant: AcouSort AB, 223 81 Lund (SE)
(72) Inventor: STECKEL, André Gugele, 2800 Kongens Lyngby (DK); BRUUS, Henrik, 2100 København Ø (DK); LAURELL, Thomas, 224 67 Lund (SE)
(74) Representative: Brann AB

(57) **Abstract**

An acoustofluidic device comprising: - a substrate in which a microfluidic cavity is positioned, and - a thin film ultrasound transducer provided in acoustic contact with at least a part of one surface of the substrate for transferring ultrasonic vibrations to the substrate and causing the substrate to vibrate, wherein the thin film ultrasound transducer preferably has a thickness of less than 100 µm, such as less than 50 µm, such as less than 5 µm, such as less than 2 µm, is provided. A method of performing an acoustofluidic operation and a method of manufacturing an acoustofluidic device are also provided.

## Description

### Technical field

The technology proposed herein relates generally to the field of acoustofluidics in which ultrasound is used to actuate waves in liquids and suspensions for interacting with the liquids or different types of particles in the liquids and suspensions, for example for performing inter alia separation and sorting of the particles or mixing of liquids or suspensions. The technology proposed herein particularly relates to devices and methods for performing such acoustofluidic operations using thin film actuators instead of commonly used piezoelectric bulk ultrasound transducers.

### Background

Acoustofluidics generally refer to using sound to affect liquids or particles in liquids or suspensions. Acoustofluidics used to affect particles is generally termed acoustophoresis. Acoustophoresis has been used inter alia for separating different types of cells in suspensions such as separating blood cells from plasma or for separating and collecting circulating tumor cells from blood. Generally, an acoustofluidic device comprises a microfluidic cavity such as a microfluidic flow channel fashioned in a substrate. The suspension is pumped through the flow channel under laminar flow conditions, or alternatively is stationary in the flow channel. An ultrasound transducer, particularly a piezoelectric element, is attached to the substrate and actuated to produce an ultrasonic vibration in the substrate in the range of about 1-10 Mhz. Provided that the dimensions, in particular height or width, of the flow channel is properly matched with the frequency of the ultrasonic vibration, a standing wave may appear in the channel. This standing wave exerts a force on the particles in the suspension dependent on the acoustic contrast of each individual particle as determined by the properties of each particle relative to those of the suspending liquid in the suspension, and thus particles will be forced to move, dependent on the acoustic contrast, towards or away from the pressure node(s) of the standing wave.

Applications include, as stated above, separation, sorting, trapping and other manipulations of the particles. More general applications of acoustofluidics involve mixing of liquids. Generally, an acoustofluidic device can be used of both general acoustofluidic operations such as mixing of liquid, as well as for acoustophoretic operations such as manipulating particles.

Generally, as mentioned above, a piezoelectric element, i.e., a solid piece of a piezoelectric material, is used to transduce or convert an input electric signal into the ultrasonic vibration.

Piezoelectric materials inter alia include crystalline materials having non-centrosymmetric crystal structure such as langasite (La₃Ga₅SiO₁₄), gallium orthophosphate (GaPO₄) and lithium niobate (LiNbO₃) and ferroelectric ceramics with randomly oriented grains such as lead zirconate titanate (PZT) with the formula (Pb[ZrₓTi₁₋ₓ]O3 with 0 ≤ x ≤ 1) which is a commonly used piezoelectric ceramics, and Sodium potassium niobate ((K,Na)NbO₃).

To obtain an ultrasound transducer for use in acoustophoresis, a suitably sized solid piece of piezoelectric material is obtained and provided with electrodes for conducting the electrical signal to the piezoelectric material. Both obtaining the suitably sized piece and providing the electrodes may be time consuming and labor intensive.

As one example, a larger piece of piezoelectric material may need to be cut or otherwise machined so as to obtain a piece of suitable dimensions. The suitable dimensions are dimensions such that the natural resonance frequencies of the piece, as governed by its dimensions (e.g., width, length, height), match or otherwise are suitable for the frequency at which it is intended to actuate the ultrasound transducer. Such ultrasound transducers are termed bulk ultrasound transducers in that resonance occurs in the bulk of the transducer itself. This is also referred to as bulk acoustic waves (BAW).

Additionally, once the ultrasound transducer has been produced from the piece of piezoelectric material, it generally needs to be manually or semi-manually attached to the acoustofluidic device, a process which also may take time and be labor intensive, or which may lead to low reproducibility when manufacturing the acoustofluidic device.

The size and generally cuboid or wedge-shaped form of the ultrasound transducer may further add bulk to the acoustofluidic device, which otherwise tend to be in chip form, i.e., flat, or capillary form, i.e., elongated. This may inconvenience the integration of the acoustofluidic device into for example a larger analysis system.

A further difficulty associated with using bulk ultrasound transducers in acoustofluidic devices is that the bulk and inherent resonances in the bulk ultrasound transducer significantly affects the resonance conditions of the substrate to which it attached. This effect depends on the size and weight relation between the transducer and the substrate, and may therefore result in unexpected effects when actuating differently sized and configured substrates using the same type and size of bulk ultrasound transducer.

In summary, acoustofluidic methods and devices using conventional piezoelectric bulk ultrasound transducers generally suffer from the abovementioned disadvantages.

Thin film ultrasound transducers have been used in the field of acoustofluidics. Here thin film typically refers to material layers with a thickness in the nm to µm range, i.e., significantly thinner than bulk transducers (which typically have dimensions in the several hundreds of µm to mm range, often so as to have a half or quarter wavelength thickness resonance at the operation frequency).

As one example, Reichert et al. Lab Chip 18, 3665 (2018) discloses using a thin film ultrasound transducer provided in contact with a sample fluid via a thin flexible membrane that defined one wall of a microfluidic channel in which the sample fluid is placed. Actuation of the thin film ultrasound transducer mechanically pressurizes the sample fluid. Also, WO0228523 mentions using a thin film ultrasound transducer, which however is placed directly inside a microfluidic channel. In these cases the substrate, in which the channel is provided, is not caused to vibrate by the thin film ultrasound transducer.

Finally, WO04030800 generally refers to an ultrasound transducer attached to an outside surface of a substrate as possibly being a thin film ultrasound transducer. However, as the document further states that the transducer should have sufficient thickness so that resonance occurs within the transducer itself, this cannot be considered a thin film ultrasound transducer.

### Objects

The technology proposed herein aims at obviating or overcoming the aforementioned disadvantages stemming from the use of conventional bulk ceramic ultrasound transducers.

A primary object of the technology proposed herein is therefore to provide an acoustofluidic device comprising a thin-film ultrasound transducer.

A further object of the technology proposed herein is to provide a method of performing an acoustofluidic operation in an acoustofluidic device comprising a thin-film ultrasound transducer.

It is yet another object of the technology proposed herein to provide a method of producing an acoustofluidic device comprising a thin-film ultrasound transducer.

### Summary

At least one of the abovementioned objects or at least one of the further objects which will become evident from the below description, are according to a first aspect of the technology proposed herein achieved by an acoustofluidic device comprising:
- a substrate in which a microfluidic cavity is positioned, and
- a thin film ultrasound transducer provided in acoustic contact with at least a part of one surface of the substrate for transferring ultrasonic vibrations to the substrate and causing the substrate to vibrate, wherein the thin film ultrasound transducer preferably has a thickness of less than 100 µm, such as less than 50 µm, such as less than 5 µm, such as less than 2 µm.

At least one of the abovementioned objects or at least one of the further objects which will become evident from the below description, is further, according to a second aspect of the technology proposed herein, achieved by a method of performing an acoustofluidic operation, comprising the steps of:
a. providing an acoustofluidic device comprising:
   - a substrate in which a microfluidic cavity is positioned, and
   - a thin film ultrasound transducer provided in acoustic contact with at least a part of one surface of the substrate for transferring ultrasonic vibrations to the substrate and causing the substrate to vibrate,
   wherein the acoustofluidic device preferably is an acoustofluidic device according to the first aspect of the technology proposed herein,
b. actuating the thin film ultrasound transducer at a frequency f that corresponds to an acoustic resonance peak of the substrate including the microfluidic cavity filled with a liquid or a liquid suspension, and
c. providing the liquid suspension in the microfluidic cavity to perform the acoustofluidic operation on the liquid or the liquid suspension.

Accordingly, the technology proposed herein is based on the discovery by the present inventors that a thin film ultrasound transducer surprisingly can be used in practice to actuate a substrate similar to how a bulk ultrasound transducer is used. This however requires that the thin film ultrasound transducer is driven at a frequency f that corresponds to an acoustic resonance peak of at least the substrate including the microfluidic cavity filled with a liquid or liquid suspension. In particular, it is the interface formed by the differing acoustic impedance of the substrate and the surrounding air at the outer surface of the substrate that causes reflection of the sound so that resonance in the whole of the substrate is obtained. This is also called whole body resonance. This resonance could be a one- or two-dimensional standing wave, but is preferably a three-dimensional volume resonance of the whole substrate including the microchannel that may or may not be possible to describe as a one- or two-dimensional resonance or superposition of such resonances.

This differs from conventional bulk ultrasound transducers which generally are actuated at a resonance frequency of the microfluidic cavity *per se,* i.e., without regard to the acoustic resonance peaks of the whole substrate. Such bulk ultrasound transducers further, due to their millimeter dimensions, significantly affect the resonance of the acoustofluidic device itself. In contrast, the thin film ultrasound transducers used in the technology proposed herein typically have thicknesses of less than 100 µm, such as less than 10 µm, and therefore provide only a negligible influence on the resonance of the acoustofluidic device itself. This further differs from previous attempts at using thin film ultrasound transducers in acoustofluidic devices.

According to the technology proposed herein the thin film ultrasound transducer is in acoustic contact with at least a part of one surface of the substrate for transferring ultrasonic vibrations to the substrate and causing the substrate to vibrate. In contrast, the thin film ultrasound transducers used in prior attempts have been provided in direct contact with the fluid in the channel in those substrates, or have been used to actuate a thin membrane in contact with the fluid.

As noted in Example 1, the technology proposed herein provides that an acoustofluidic device with a thin film ultrasound transducer having a thickness in the µm range can provide acoustofluidic effects in the microfluidic cavity that are of the same order of magnitude as those obtained by conventional bulk ultrasound transducers having dimensions in the mm range, however without the disadvantages of the latter.

The technology proposed herein thus offers several advantages for acoustofluidic devices and acoustofluidic methods: it enables higher reproducibility of acoustofluidic devices (due to precise microfabrication of the thin film ultrasound transducers, such as in a clean room), dissipative damping due to the ultrasound transducer much less affects the resonance quality of the entire acoustofluidic device, and it may further enable mass fabrication of thin film ultrasound transducers and acoustofluidic devices. In addition, the acoustofluidic device is not particularly sensitive to off-sets of the thin film ultrasound transducer or the microfluidic cavity within the substrate.

The acoustofluidic operation generally involves affecting a liquid or suspension, including any particles in the liquid or suspension, and may comprise or consist of an acoustophoretic operation which may include one or more of focusing, i.e. causing particles to move to discrete areas of the microfluidic cavity, trapping, i.e. retaining particles in the microfluidic cavity, separating, i.e. causing different particles (which particle differ in size and/or acoustic contrast compared to the liquid in the microfluidic cavity) to move in different directions and/or with different speeds.

In the context of the technology proposed herein acoustofluidic device is to be understood as encompassing acoustophoretic device, acoustophoresis chip, and acoustophoresis device.

In the context of the technology proposed herein the thickness of the thin film ultrasound transducer is the thickness of the material layer that provides the ultrasound vibrations. As an example, where the thin film ultrasound transducer comprises a layer of piezoelectric or electrostrictive material, the thickness of any electrode layer or layers used to deliver an electric signal to the layer of piezoelectric or electrostrictive material are not included in the thickness of the thin film ultrasound transducer.

The substrate may be made from a number of different materials including glass, metal, ceramics, and silicon. It is further contemplated within the context of the technology proposed herein that the substrate may be made from polymeric materials, in particular plastics such as cyclic olefin copolymer (COP), cyclic olefin polymers (COC), polycarbonate (PC), polypropylene (PP) poly(methyl methacrylate) (PMMA), polystyrene (PS), and Polyether Ether Ketone (PEEK).

The substrate may have different shapes, lengths, heights, and widths provided that there exists a resonance peak corresponding to resonance in the substrate at a frequency in the ultrasound range, preferably in the range of 0.1 to 20 MHz, more preferably in the range of 0.8 to 8 MHz, most preferably in the range of 1 to 5 MHz.

Typically, the substrate has a bottom surface, an opposing top surface, two opposing side surfaces, and two opposing end surfaces. The length, height and width of the substrate are typically in the range of 10-100 mm (length) 0.5 to 3 mm (height), and 1-10 mm (width). The substrate may for example be as a capillary.

The microfluidic cavity may run along at least a part of the substrate and may be provided with inlets and outlets at its opposite ends. The microfluidic cavity may in particular comprise a microfluidic channel. The microfluidic cavity may have a floor, a ceiling, and two opposing side walls. Typically, the microfluidic cavity will have a rectangular or substantially rectangular cross section, although other shapes of cross section are possible. The width of the microfluidic cavity is typically from 0.1 to 1 mm and the height 0.05 to 0.5 mm, depending on the size of any particle that is to pass through the microfluidic cavity. It is to be understood that a liquid or suspension in the microfluidic cavity may, or may not, flow through the cavity.

In some embodiments the width the microfluidic cavity can be up to 4 mm and the height of microfluidic cavity can be up to 2 mm.

The microfluidic cavity is positioned in the substrate such that the resonance in the substrate gives rise to acoustic forces on any particle having a different acoustic contrast than the liquid the particle is suspended in. The substrate may be formed in one piece. Typically, however, the substrate is fashioned from two parts so that the cavity may be easily implemented as a trough or groove in one of the parts whereafter the other part is placed as a lid to seal the trough or groove to form the cavity. The lid is not a membrane because it is thick enough that, if the thin film ultrasound transducer is attached to it, the lid transfers the ultrasound vibration to the rest of the substrate so that the whole substrate vibrates. Typically, the lid has a thickness that is at least as thick as the minimum dimension of the microfluidic cavity, or at least 1/10 of the minimum outer dimension of the substrate. The cavity is not provided in the thin film ultrasound transducer.

The cavity may further have different dimensions at different positions along its length. The cavity may further branch into plural cavities, or plural cavities may join into one cavity, at different positions along its length.

Typically, the microfluidic cavity has at least one minimum dimension, such as a width or height, and the thickness of the thin film ultrasound transducer is smaller than the at least one minimum dimension.

Preferably the substrate has a minimum dimension, such as a width or height, and the thickness of the thin film is less than 1/10 or more preferably less than 1/100 of the minimum dimension.

The thin film ultrasound transducer typically makes up less than 0.001 of the total volume of the acoustofluidic device.

The thin film ultrasound transducer may comprise a layer of piezoelectric material to which a first and second electrodes have been connected in order to supply electric energy to the piezoelectric material to cause it to vibrate.

Preferably the thin film ultrasound transducer comprises a layer of piezoelectric or electrostrictive material placed between a first electrode layer defining a first side of the thin film ultrasound transducer and a second electrode layer defining a second side of the thin film ultrasound transducer, and wherein the first side of the thin film ultrasound transducer is provided in acoustic contact with the at least one part of the one surface of the substrate. The first and second electrode layers should be made of an electrically conductive material such as a metal, for example silver or gold.

The thin film ultrasound transducer may be placed at different positions on the substrate. Where the substrate comprises a base substrate in which the cavity is formed as a groove or similar, and wherein a lid substrate is attached to the base substrate to cover and together with the base substrate define the cavity, then the thin film ultrasound transducer may preferably be attached to the base substrate. Alternatively, the thin film ultrasound transducer may be attached to the lid substrate. However, in each case the thin film ultrasound transducer is positioned so that it can cause the whole substrate to vibrate.

Further, the lid substrate or the base substrate may be configured with different dimensions, such that for example the lid substrate has a larger area than the base substrate, such that the base substrate only is attached to a part of a surface of the lid substrate, or vice versa.

The at least one thin film ultrasound transducer may be in acoustic contact with the substrate by being in direct physical contact, or by being in indirect physical contact via for example an acoustically conducting material, such as an electrode or electrode layer that is used to conduct an electric signal to the thin film ultrasound transducer.

Preferably the thin film ultrasound transducer is provided in acoustic contact with a majority, such as all, of the one surface. Alternatively the thin film ultrasound transducer is provided in acoustic contact with a part of the one surface. It may be preferably to provide the thin film ultrasound transducer so that at least a portion of the microfluidic cavity can be observed through the one surface.

Preferably the thin film ultrasound transducer is deposited onto the surface of the substrate. Alternatively, the thin film ultrasound transducer is separate from the substrate and attached to the surface of the substrate by adhesive.

The thin film ultrasound transducer may be deposited by first depositing a first electrode or electrode layer onto the surface. Then a layer of piezoelectric or electrostrictive material is deposited onto the first electrode or electrode layer. Finally, a second electrode or electrode layer deposited onto the layer of piezoelectric or electrostrictive material. As above, the first and second electrode or electrode layers should be made of an electrically conductive material such as a metal, for example platinum, silver, or gold. Additional layers may be included for facilitating attachment of the thin film ultrasound transducer to the substrate. As an example, a first layer of titanium (such as 1 nm) may be deposited on the substrate before an electrode or electrode layer is deposited. The thickness of the electrodes or electrode layers may vary. The electrode layers may for example have a thickness of 50 to 200 nm, such as 70 to 150 nm, such as 80-100 nm. Generally, the electrodes or electrode layers are therefore thinner than the material providing the ultrasound vibrations, i.e., typically the piezoelectric or electrostrictive material.

Typically, the thin film ultrasound transducer comprises a piezoelectric or electrostrictive material which is caused to vibrate by an electric drive signal. However, it is further contemplated within the context of the technology proposed herein that also other types of ultrasound transducer materials, such as magnetostrictive materials (which change dimension in magnetic fields) and thermoacoustic materials (which emit acoustic vibrations in response to temperature changes) can be used. It is further contemplated within the context of the technology proposed herein that the thin film ultrasound transducer could employ an electrostatic function, whereby vibrations are caused by varying the electrostatic attraction and/or repulsion between electrodes of the thin film ultrasound transducer.

Preferably the thickness of the thin film ultrasound transducer is less than 10 µm, more preferably 5 µm or less, such as 0.01 to 5 µm, more preferably 3 µm or less, such as 0.5 to 3 µm, more preferably 2 µm or less, such as 0.5 to 2 µm, such as 0.5 to 1.5 µm, such as 1 µm or less than 1 µm. These thicknesses are typically less than ¼ of the wavelength, in the thin film ultrasound transducer material, for the acoustofluidic device resonance frequency used when actuating the acoustofluidic device to perform an acoustofluidic operation.

These are typical thicknesses for thin films. However, the thin film ultrasound transducer may be significantly thinner, such as down to one or more atomic or molecular monolayers. Generally, a thinner thin film ultrasound transducer may be easier to manufacture, whereas a thicker thin film ultrasound transducer may provide a stronger acoustofluidic effect in the microfluidic cavity.

Preferably the acoustofluidic device comprises:
- a further thin film ultrasound transducer provided in acoustic contact with at least a part of the one surface of the substrate for transferring ultrasonic vibrations to the substrate and causing the substrate to vibrate.

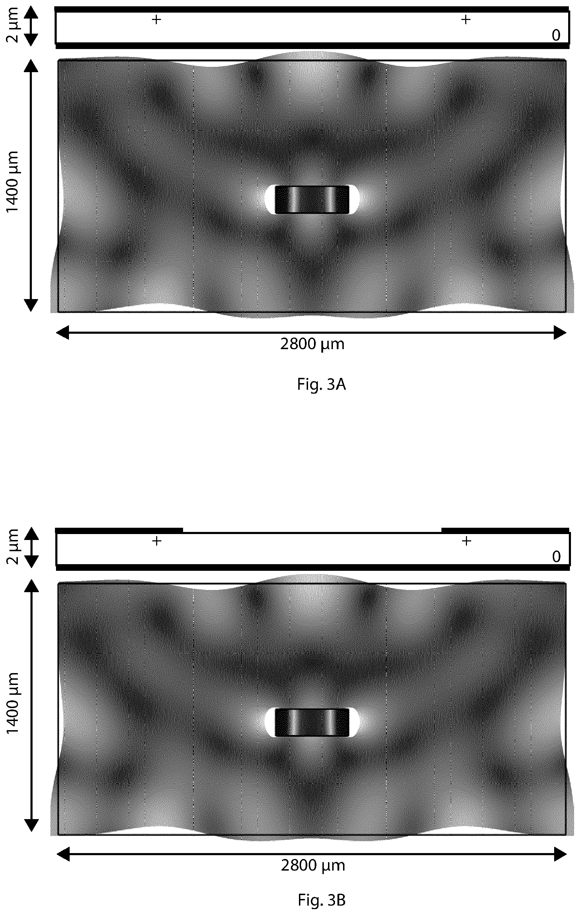

This is advantageous in that, as seen in example 1, Fig. 3B the use of two or more thin film ultrasound transducers, separate or defined by electrode segments, may increase the strength of the displacement field and thereby the pressure effects in the microfluidic cavity, thus leading to more efficient and effective acoustofluidic operations.

The further thin film ultrasound transducer may be configured with the same, or different, dimensions and materials as described herein for the thin film ultrasound transducer.

The thin film ultrasound transducer and the further thin film ultrasound transducer may be termed first thin film ultrasound transducer and second thin film ultrasound transducer.

The thin film ultrasound transducer and the further thin film ultrasound transducer may be provided as two separate thin films on the one surface. Preferably the two separate thin films are placed spaced apart from each other, more preferably at or along opposite borders of the one surface.

The thin film ultrasound transducer and the further thin film ultrasound transducer are preferably in acoustic contact with the same one surface of the substrate. The thin film ultrasound transducer and the further thin film ultrasound transducer are preferably positioned side by side in acoustic contact with the one surface of the substrate.

The one surface of the substrate may be any surface of the substrate but is typically the bottom surface or the top surface, and preferably the bottom surface.

It is however contemplated within the context of the technology proposed herein that the thin film ultrasound transducer and the further thin film ultrasound transducer are provided in contact with different surfaces of the substrate.

The thin film ultrasound transducer and the further thin film ultrasound transducer may be provided with one electrode in common.

Accordingly, at least one of the first and second electrode layers may be divided into at least two portions, thereby defining two separate electrodes, which two electrodes, together with the layer of piezoelectric or electrostrictive material and the electrode defined by the other of the first and second electrode layers, define the thin film ultrasound transducer and the further thin film ultrasound transducer as having a common layer of piezoelectric or electrostrictive material and one common electrode layer.

For simplicity, it is preferred that it is the second electrode layer that is divided.

If the thin film ultrasound transducer and the further thin film ultrasound transducer are to be actuated in phase, then it is preferred that the two portions of the divided electrode layer are spaced apart on the layer of piezoelectric material such that a portion, such as 20-80%, such as 25-75&, such as 40-60%, such as 50%, of the piezoelectric material is not covered by the two portions of the divided electrode.

The divided electrode layer may be divided into further portions, for defining further thin film ultrasound transducers.

The positions and extensions of the thin film ultrasound transducer and the further thin film ultrasound transducer may be advantageously configured so as to allow the microfluidic cavity to be observed or analyzed through the one surface, wherein preferably the thin film ultrasound transducer and the further thin film ultrasound transducer are positioned spaced apart at or along opposite borders of the one surface so as to allow the microfluidic cavity to be observed or analyzed through the part of the one surface between the thin film ultrasound transducer and the further thin film ultrasound transducer.

Such an arrangement of the thin film ultrasound transducer and the further thin film ultrasound transducer may be provided by providing a first electrode layer having a U or H shape, or other shape defining two spaced apart but partially joined first electrodes, and providing similarly shaped but separate layers of piezoelectric material and second electrode layers onto the first two spaced apart but partially joined first electrodes. Preferably the thin film ultrasound transducer and the further thin film ultrasound transducer are then actuated in antiphase, i.e., antisymmetric. Thus, two drive circuits, or one drive circuit having two channels, may be used to drive the thin film ultrasound transducer and the further thin film ultrasound transducer.

The thin film ultrasound transducer may comprise any type of piezoelectric or electrostrictive material. It is however preferred to use a crystalline material having a non-centrosymmetric crystal structure such as langasite (La₃Ga₅SiO₁₄), gallium orthophosphate (GaPO₄) and lithium niobate (LiNbO₃), lithium tantalate (LiTaO₃), or a ferroelectric ceramic with randomly oriented grains such as lead titanate (PbTiO₃), potassium niobate (KNbO₃), sodium tungstate (Na₂WO₃) lead zirconate titanate (PZT) with the formula (Pb[ZrₓTi₁₋ₓ]O₃ with 0 ≤ x ≤ 1), as well as lead-free ceramics such as sodium potassium niobate ((K,Na)NbO₃), bismuth ferrite (BiFeO₃), sodium niobate (NaNbO₃), barium titanate (BaTiO₃), bismuth titanate (Bi₄Ti₃O₁₂), and sodium bismuth titanate (NaBi(TiO₃)₂), for example.

Further materials include lead magnesium niobate (PMN), lead magnesium niobate-lead titanate (PMN-PT), and lead lanthanum zirconate titanate (PLZT).

Other possible materials include molybdenum disulfide which exhibits piezoelectricity also in monolayer form.

The thin film ultrasound transducer may for example comprise a piezoelectric or electrostrictive material selected from the group consisting of zinc oxide, aluminum nitride, scandium-doped aluminum nitride, cerium oxides, and lead-zirconate-titanate.

It may be preferable to exclude lead-zirconate-titanate from the piezoelectric or electrostrictive material comprised by the thin film ultrasound transducer so as to avoid using piezoelectric or electrostrictive material containing lead (Pb) .

The at least one thin film ultrasound transducer may be actuated by providing an electric signal, such as a sine or square wave signal to the thin film ultrasound transducer in order to force the ultrasound transducer to vibrate at or near the frequency of the actuation signal. Actuating the at least one thin film ultrasound transducer at the frequency f is further to be understood as encompassing supplying ultrasound energy at the frequency f to the substrate.

The frequency f is typically in the range of 0.1 to 20 MHz. The voltage of the signal (peak-peak) may be 1 - 30 Vpp, such as 10-20 Vpp. The higher the voltage, the stronger the acoustofluidic effect. The maximum voltage used is limited by the breakdown voltage V/pm thickness of the piezoelectric or electrostrictive material used. The breakdown voltages depend on the piezoelectric or electrostrictive materials used and can generally be in the range of 0.5 to 20, such as 10 to 20 Vpp/µm. The maximum voltage is also limited by the heating of the material during actuation.

Typically, the frequency that results in resonance in the substrate is different from the frequency that would result in resonance in the microfluidic cavity when a bulk ultrasound transducer is used.

The signal may be provided by a drive circuit such as a function generator. The drive circuit may be separate from the acoustofluidic device.

The acoustofluidic device may further comprise:
- a drive circuit configured to actuate the thin film ultrasound transducer at a frequency f that corresponds to an acoustic resonance peak of the substrate including the microfluidic cavity filled with a liquid or liquid suspension, and wherein the drive circuit preferably further comprises an inductor connected in series with the thin film ultrasound transducer.

In the context of the technology proposed herein corresponds is to be understood as preferably, but not exclusively, relating to an exact match of the frequencies - it is contemplated that a satisfactory actuation of the substrate will be possible even where the frequency f differs from the resonance peak by no more than 30%, preferably no more than 20%, more preferably no more than 10%, and most preferably no more than 1% such as no more than 0.1%.

The acoustic resonance peak is the frequency where the acoustic energy in the substrate reaches a maximum. There may be several acoustic resonance peaks for a given substrate.

The resonance peak should at least correspond to a resonance peak of the substrate in its entirety. Preferably the resonance peak should correspond to the resonance of the substrate including the microfluidic cavity including the liquid inside the cavity.

This resonance could be a one- or two-dimensional standing wave, but preferably the acoustic resonance peak corresponds to a three-dimensional volume resonance of the whole substrate including the microfluidic cavity that may or may not be possible to describe as a one- or two-dimensional resonance or superposition of such resonances. Typically, the acoustic resonance peak corresponds to three-dimensional volume resonance in the substrate including the microfluidic cavity, which three-dimensional volume resonance cannot be described as a one- or two-dimensional resonance in the substrate, and wherein preferably the frequency f does not correspond to a resonance frequency of the microfluidic cavity alone.

As seen in Example 3, the introduction of an inductor, preferably a tunable inductor, in the drive circuit in series with the thin film ultrasound transducer can increase the power delivery to the substrate for a given resonance frequency at a given actuation (drive) voltage.

The liquid or liquid suspension may be provided in the microfluidic cavity by pumping, suction, etc. The liquid or liquid suspension may be flowed through the microfluidic cavity or injected and stopped in the cavity.

The liquid suspension may be a disperse fluid such as undiluted or diluted whole blood, intracellular fluid, interstitial fluid, synovial fluid, peritoneal fluid, urine, yeast cell cultures, bone marrow, stroma, dissociated cells from normal or cancerous tissue, milk. The liquid suspension may comprise particles such as red blood cells, white blood cells, platelets, cancer cells, bacterial cells, viruses, yeast cells, algae, pollen, extracellular vesicles such as microvesicles and exosomes, dust particles, silica particles, magnetic particles, and polymer particles.

The acoustofluidic operation may comprise or be followed by a measurement or optical observation or interrogation in the microfluidic cavity.

Preferably the acoustofluidic device comprises the further thin film ultrasound transducer, and the drive circuit is further configured to actuate the thin film ultrasound transducer and the further thin film ultrasound transducer at the frequency f in phase, or preferably out of phase such as in antiphase, to each other. Correspondingly, in a preferred embodiments of the method:
- step a comprises providing an acoustofluidic device comprising the further thin film ultrasound transducer, and
- step b comprises actuating the thin film ultrasound transducer and the further thin film ultrasound transducer at the frequency f in phase, or preferably out of phase such as in antiphase, to each other.

In phase means that the thin film ultrasound transducer and the further thin film ultrasound transducer are actuated in concert, i.e., so as to be actuated without any phase shift between the two signals to the two thin film ultrasound transducers. As seen in Example 1, in-phase actuation of two thin film ultrasound transducer provides a stronger acoustofluidic effect in the cavity than actuation of a single thin film ultrasound transducer.

Out of phase is to be understood as any phase shift between the two signals to the two thin film ultrasound transducers. Preferably however the phase shift is 90° to 270°, such as preferably 135° to 225°, such as preferably 170° to 190°, most preferably 180° (antiphase).

As seen in Example 1, actuating the two thin film ultrasound transducers in antiphase provides even stronger acoustofluidic effects in the cavity than in-phase actuation.

The acoustofluidic operation may be any acoustofluidic or acoustophoretic operation such as mixing of fluids or suspensions, pumping, sonoporation and sonolysis, or affecting cells or particles in a liquid or suspension.

The acoustofluidic operation may comprise or consist of an acoustophoretic operation encompassing manipulating cells or other particles, e.g., comprising focusing cells or other particles, suspended in a suspension within the microfluidic cavity, towards one or more discrete areas of the microfluidic cavity.

Focusing is to be understood as encompassing moving.

The substrate may additionally comprise a further microfluidic cavity, the further microfluidic cavity being positioned so that that an acoustic force arises, due to resonance in the substrate preferably including the microfluidic cavity and the further microfluidic cavity, on a target particle or liquid in the further microfluidic cavity, the acoustic force being the same, or different, from an acoustic force arising on a target particle in the microfluidic cavity.

These embodiments utilize the fact that the technology proposed herein takes into account the resonance in the entire substrate. In particular the acoustic force may be dependent on the position of a cavity within the substrate, thus providing for obtaining different acoustic forces in different parts of the substrate.

The target particle is the particle or particles which should be moved or otherwise affected by the acoustofluidic operation.

The further microfluidic cavity may have the same dimensions and configuration as described above for the microfluidic cavity.

The technology proposed herein further provides a new principle of designing and manufacturing of acoustofluidic devices comprising a thin film ultrasound transducer. At least one of the abovementioned objects, or at least one of the further objects which will become evident from the below description, is therefore, according to a third aspect of the technology proposed herein, achieved by a method of producing an acoustofluidic device for performing an acoustofluidic operation, the acoustofluidic device comprising a substrate in which a microfluidic cavity is provided, comprising the steps of:
a. determining, experimentally, or by calculation or simulation, the acoustic resonances of the substrate for each of a plurality of different combinations of parameter values of substrate parameters, the substrate parameters including substrate material, substrate dimensions, microfluidic cavity dimensions, microfluidic cavity position within the substrate, properties of a liquid in the microfluidic cavity, position for at least one, preferably two, thin film ultrasound transducer in acoustic contact with at least a part of one surface of the substrate, and actuation frequency f, and
b. selecting, among the plurality of different combinations of the parameter values of the substrate parameters, a substrate material M, a set of substrate dimensions D_{S}, a set of microfluidic cavity dimensions D_{C}, a microfluidic cavity position P_{C} within the substrate, properties of the liquid L in the microfluidic cavity, a position P_{U} for the at least one, preferable two, thin film ultrasound transducer, and an actuation frequency f, which yield acoustic resonance within the substrate including the microfluidic cavity for performing the acoustofluidic operation, and
c. manufacturing the acoustofluidic device made out of the substrate material M having the substrate dimensions D_{S} and being provided with a microfluidic cavity having the microfluidic cavity dimensions D_{C} and the microfluidic cavity position P_{C} within the substrate.

As discussed above for the acoustofluidic device according to the first aspect of the technology proposed herein, the use of thin film ultrasound transducers for actuating a substrate involves taking into account resonance in the whole of the substrate, in particular taking into account the dimensions of the substrate and the position of the microfluidic cavity within the substrate.

In the context of the technology proposed herein producing is to be understood as encompassing designing and/or constructing. The method according to the third aspect of the technology proposed herein may alternatively comprise steps a and b, whereby the selected parameters, i.e. the substrate material (or a combination of materials) M, the set of substrate dimensions D_{S}, the set of microfluidic cavity dimensions D_{C}, the microfluidic cavity position P_{C} within the substrate, the properties of the liquid L in the microfluidic flow channel, the position P_{U} for at least one thin film ultrasound transducer, and the actuation frequency f, define design parameters for designing the acoustofluidic device.

Preferably the determining in step a is performed by calculation or simulation.

The calculation or simulation preferably comprises simulating the acoustic resonances in at least a two-dimensional, preferably a three-dimensional, model of the substrate. The parameter values may all be varied over a range of possible values, typically however some of the values are set, such as for example substrate material and substrate dimensions. Typically, therefore it is the frequency that is varied in order to find a frequency giving rise to resonance in the substrate.

The method according to the third aspect of the technology proposed herein may also be performed for substrate parameters which include several cavities each having its own set of microfluidic cavity dimensions, microfluidic cavity positions within the substrate, and properties of a liquid in the microfluidic cavities.

Thus, the method may be used to manufacture or design acoustofluidic devices having more than one channel for performing more than one acoustofluidic operation.

In step a a plurality of more than one actuation frequency may be included in determining the acoustic resonances of the substrate. Thus, in step b, more than one actuation frequency f may be selected in order to generate a superposition of the acoustic fields generated by each frequency.

This may for example be useful where determining the acoustic resonances of the substrate reveals that there are several useful resonance frequencies or when a superposition of them is desired.

Where in step a a plurality of positions for two thin film ultrasound transducers is included in determining the acoustic resonances of the substrate, then a plurality of phase differences between the two thin film ultrasound transducers may be included in determining the acoustic resonances of the substrate, and a phase difference between the two thin film ultrasound transducers may be selected in step b.

In preferred embodiments of the method according to the third aspect of the technology proposed herein the method further comprises, as a part of step c or as a step d after step c, the step of attaching at least one thin film ultrasound transducer to the substrate at the position P_{U} for the at least one ultrasound transducer.

In preferred embodiments of the method according to the third aspect of the technology proposed herein simulation is used in step a, the simulation using as boundaries the substrate/air interface at the outer surfaces of the substrate and the substrate/liquid interface at walls of the microfluidic cavity.

In preferred embodiments of the method according to the third aspect of the technology proposed herein:
- step a further comprises determining the acoustic force on a target particle throughout the substrate for each of the plurality of different combinations of parameter values of substrate parameters, and
- step b further comprises determining the set of microfluidic cavity dimensions D_{C} and the microfluidic cavity position P_{C} within the substrate so that the microfluidic cavity at least partly delimits a region of the substrate in which the acoustic force on the target particle is suitable for performing the acoustofluidic operation.

Alternatively, the acoustic force on the target particle is determined throughout the microfluidic cavity for each of the plurality of different combinations of parameter values of substrate parameters.

Generally, determining the acoustic force on a target particle is associated with producing an acoustofluidic device for performing an acoustophoretic operation. For producing an acoustofluidic device for performing a general acoustofluidic operation, the target particle may be replaced by a target liquid element.

Step a and b may be performed without considering the microfluidic cavity or cavities in order to find substrate resonances in cases where the cavity volume is small enough in relation to the substrate volume to have a small effect on the substrate resonances. The cavity or cavities are however preferably included in the acoustic field calculation, if the acoustic field and resulting particle forces are to be calculated in the cavity, unless the acoustic properties are similar enough for the substrate and cavity to approximate the cavity content with the substrate material.

In preferred embodiments of the method according to the third aspect of the technology proposed herein the acoustofluidic device is suitable for performing a further acoustofluidic operation in a further microfluidic cavity positioned in the substrate, and the substrate parameters of step a additionally comprise further microfluidic cavity dimensions and further microfluidic cavity position within the substrate for the further microfluidic cavity.

In preferred embodiments of the method according to the third aspect of the technology proposed herein the acoustofluidic operation and the further acoustofluidic operation are different, and step b further comprises determining a further set of microfluidic flow channel dimensions D_{C2} and microfluidic flow channel positions P_{C2} within the substrate so that the further microfluidic flow channel at least partly delimits a further region of the substrate in which the acoustic force on a target particle is suitable for performing the further acoustofluidic operation.

Further advantages with and features of the technology proposed herein will be apparent from the other dependent claims as well as from the following detailed description of preferred embodiments.

### Brief description of the drawings and detailed description

A more complete understanding of the abovementioned and other features and advantages of the technology will be apparent from the following detailed description of preferred embodiments in conjunction with the appended drawings, wherein:
- Figs. 1A-1E: shows results of a numerical simulation of the acoustofluidic response in a glass substrate.
- Figs. 2A-2B: show numerical simulations similar to Fig. 1A, but for a glass substrate with a piezoelectric Al N thin-film on the top surface actuated in antiphase vs in-phase.
- Figs. 3A-3B: show a numerical simulation similar to Fig. 2A demonstrating the enhancement of the acoustofluidic response by using two thin film ultrasound transducers driven in-phase as opposed to a single thin film ultrasound transducer.
- Figs. 4A-4B: show a numerical simulation similar to Fig. 3B but where anti-symmetric actuation, i.e., in antiphase, is used with a four times wider microchannel.
- Fig. 4C: shows a numerical simulation of how a standing-half-wave-like mode can be obtained simultaneously in each of two vertically oriented channels.
- Figs. 5A-5C: show numerical simulations similar to Fig. 2B for fixed geometry, but changing the material of the 2 µm thin-film ultrasound transducer.
- Figs. 6A-6G: show numerical simulations in 3D.
- Figs. 7A-7F: show experimental and simulated actuation of a glass acoustofluidic device using an Al N thin film ultrasound transducer.
- Fig. 8A-8B: show the impedance spectrum without, and with, an inductor added in series with the thin film ultrasound transducer
- Fig. 9A: shows the general construction of a first embodiment of an acoustofluidic device according to the first aspect of the technology proposed herein.
- Fig. 9B: shows the general construction of a second embodiment of an acoustofluidic device according to the first aspect of the technology proposed herein.
- Fig. 9C: shows the general construction of a third embodiment of an acoustofluidic device according to the first aspect of the technology proposed herein.
- Fig. 9D: shows the general construction of a fourth embodiment of an acoustofluidic device according to the first aspect of the technology proposed herein.
- Fig. 10: shows a flowchart of an embodiment of the method according to the second aspect of the technology proposed herein.
- Fig. 11: shows a flowchart of an embodiment of the method according to the third aspect of the technology proposed herein.

### EXAMPLE 1 - 2D and 3D simulation of thin film actuated pyrex acoustofluidic device.

### Overview

The generic model described in Nils R. Skov, Jacob S. Bach, Bjørn G. Winckelmann, and Henrik Bruus., 3D modeling of acoustofluidics in a liquid-filled cavity including streaming, viscous boundary layers, surrounding solids, and a piezoelectric transducer. AIMS Math. 4, 99-111 (2019) was used to demonstrate by numerical simulation for 2D cross-sections and for a fully 3D model that the novel thin-film actuation principle can generate MHz acoustofludic responses comparable to conventional systems in a single channel where suspended particles focus at the pressure nodal planes. The method furthermore allows for both odd and even wave patterns, higher throughput by allowing for wider channels and even the simultaneous excitation of waves in more than one channel. Additionally, we show that the different thin-film materials PZT, pure Al N, and scandium-doped Al N in the form Al0.6Sc0.4N all can be used in the thin film ultrasound transducer.

### Results

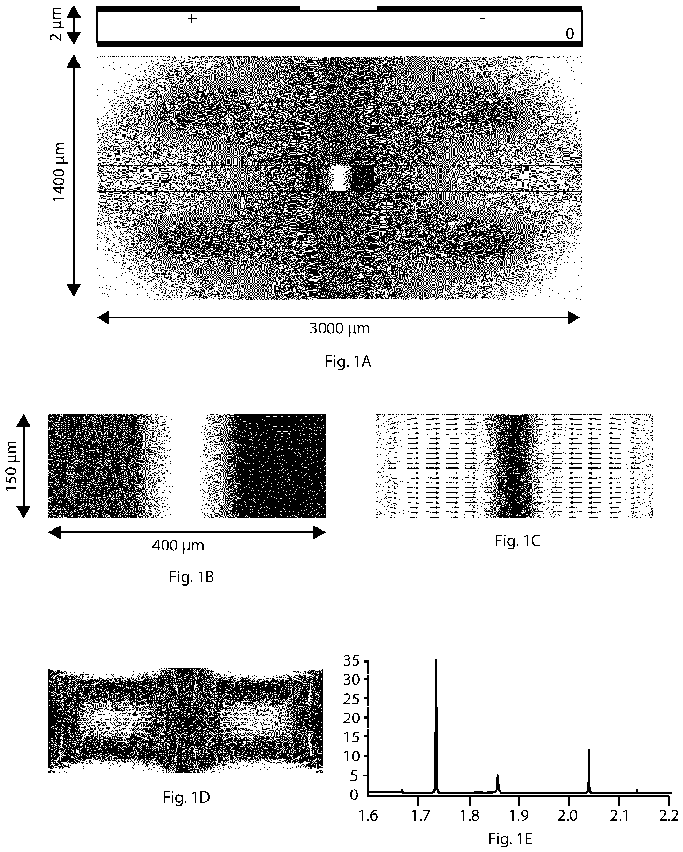

Fig. 1A shows a numerical simulation in the 2D vertical cross section of the acoustofluidic response in a 3000 µm wide and 1400 µm high glass (Pyrex) substrate when excited at the resonance frequency 1.73 MHz by a 1-Vpp ac voltage applied to a split top electrode of a 2-µm-thick piezoelectric Al_{0.6}Sc_{0.4}N thin film ultrasound transducer placed on the top surface of the substrate. The thin film ultrasound transducer has a common ground electrode layer shown closest to the substrate and marked with "0"m as well as two separate top electrode layers marked + and - when actuated in antiphase to each other. The acoustofluidic responses in the single center 150 µm x 400 µm channel match benchmark values of conventional acoustofluidic devices using bulk ultrasound transducers: MHz-frequency (see Fig. 1E showing the acoustic energy density spectrum for the water domain in the frequency range from 1.6 to 2.2 MHz used to identify the resonances of the system including a main resonance peak at 1.73 MHz), 100-kPa range pressure (see Fig 1B in which a +480kPa / -480kPa pressure was obtained in left and right regions of the channel, respectively), nm-range displacements, pN-range acoustic radiation forces (see Fig. 1C showing a radiation force of 8 pN on a 5 µm polystyrene particle suspended in water towards the center of the channel), and 10-µm/s-range acoustic streaming velocities (see Fig. 1D where a streaming velocity of 30 µm/s was obtained towards the center of the channel).

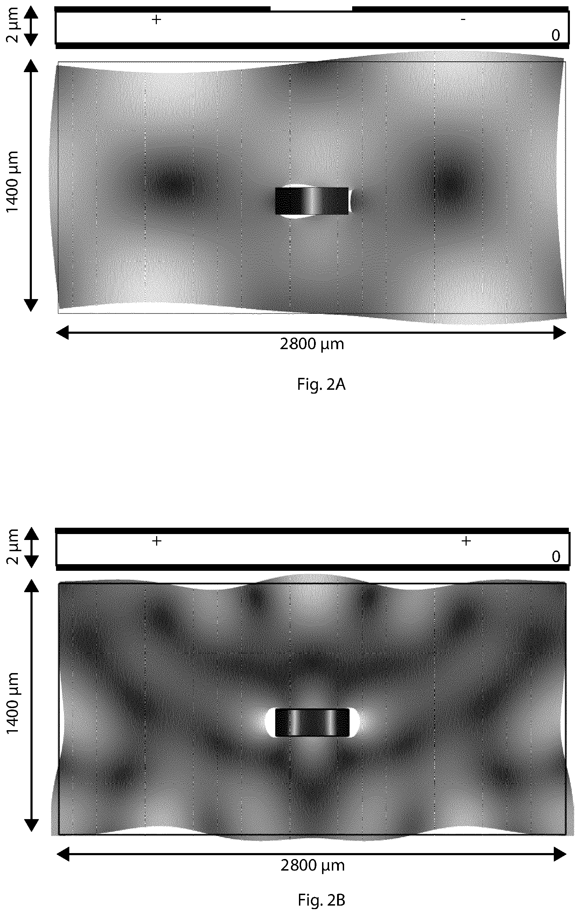

Figs. 2A-2B show numerical simulations similar to Figs. 1A, but for a 2800 µm wide and 1400 µm high Pyrex substrate with a 2-µm thick piezoelectric Aluminum nitride (Al N) thin-film on the top surface actuated/driven in antiphase vs in phase. Fig. 2A shows a split top electrode driven anti-symmetrically (see signs "+" and "-") at the resonance frequency 1.85 MHz with a single vertical pressure node in the center of the 238-kPa pressure field in the 150 µm x 400 µm channel. Fig. 2B shows a full top electrode driven symmetrically (i.e., in phase, see the two "+" signs) at its resonance frequency 3.49 MHz with two vertical pressure nodes in the 24-kPa pressure field in the 150 µm x 400 µm channel. As noted, driving two thin film ultrasound transducers (split top-electrode) in antiphase provides a stronger acoustofluidic (acoustophoretic) effect as seen in the stronger pressure field. In both cases suspended microparticles focus at the pressure nodes. The displacement field shown as the distortion of the substrate vis-à-vis its original shape (rectangular outline) has been enhanced by a factor of 100,000 throughout all figures to make the distortions at the edge visible.

Figs. 3A-3B show a numerical simulation similar demonstrating the enhancement of the acoustofluidic response by using two thin film ultrasound transducers driven in-phase as opposed to a single thin film ultrasound transducer. Accordingly, Fig. 3A shows an Al N thin film ultrasound transducer with a full top electrode whereas Fig. 3B shows the full top electrode on the 2-µm-thick piezoelectric Al N thin-film transducer being split into two electrodes each covering only 25% of the top surface of the Al N piezoelectric layer, but still driven symmetrically, i.e., in-phase as shown by the two "+" signs. The resonance mode at 3.49 MHz remains unchanged but the pressure and displacement amplitudes increase by a factor of 6 from 24 kPa to 146 kPa and from 0.1 nm to 0.6 nm, respectively, which leads to 36 times larger acoustic radiation forces on suspended microparticles in the channel.

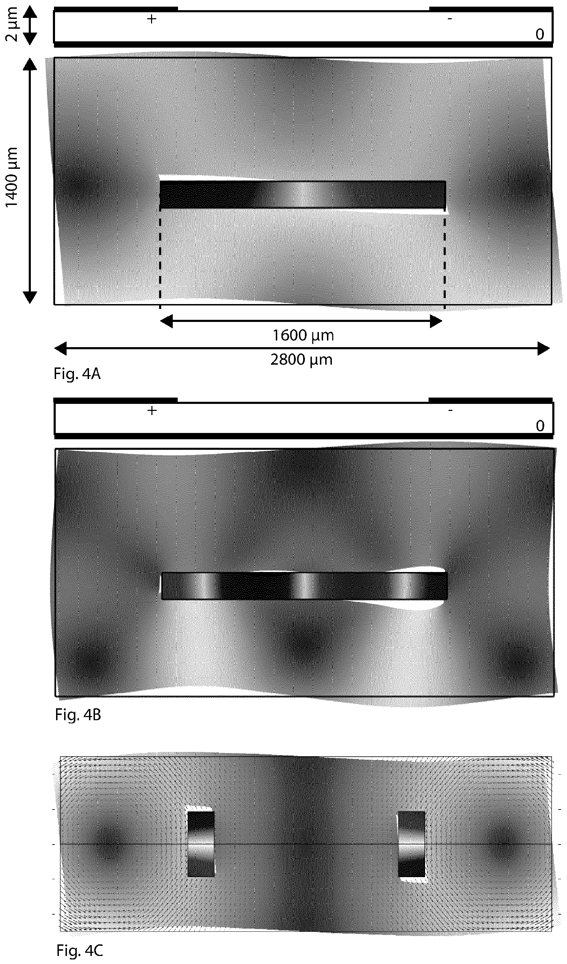

Figs. 4A-4B show a numerical simulation similar to Fig. 3B but where anti-symmetric actuation, i.e., in antiphase, is used with a four times wider microchannel having a width of 1600 µm. Fig 4A thus shows that a resonance at 0.50 MHz has one vertical pressure node for particle focusing in the center of the +- 9 kPa pressure field in the channel at a displacement field of 2.0 nm in the pyrex substrate. Fig. 4B shows that a resonance at 1.35 MHz exhibits three vertical pressure nodes in the +- 49 kPa pressure field in the channel at a displacement field of 1.2 nm in the pyrex substrate. The wider micro-channel allows for an enhanced throughput.

Fig. 4C shows a numerical simulation of how a standing-half-wave-like mode can be obtained simultaneously in each of two vertically oriented channels. The channels are 400 µm high and 150 µm wide. The substrate is 2800 µm wide and 1000 µm high pyrex block. The thin film ultrasound transducer used in the simulation is 2 µm thick with a split top electrode and actuated anti-symmetrically. The resonance frequency was 1.033 MHz, the pressure field in the channels were 13kPa, and the displacement field was 2.1 nm.

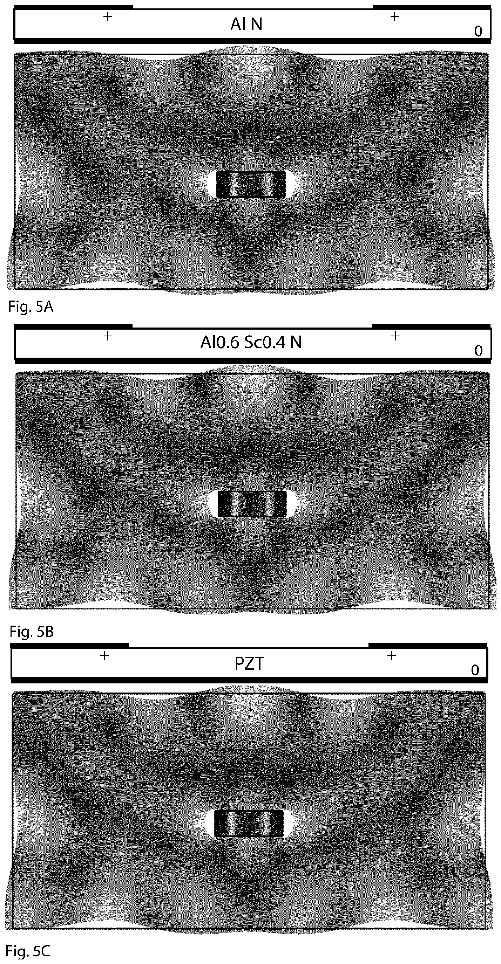

Figs. 5A-5C show numerical simulations similar to Fig. 3B for fixed geometry and symmetric actuation, but changing the material of the 2-µm thin-film ultrasound transducer weakest to strongest (1:3:10) acoustic response for a 1-Vpp applied ac voltage. Fig. 5A: Al N as in Fig. 2B providing a displacement field of 0.6 nm and a pressure field of +-146 kPa at a frequency of 3.493 MHz. Fig. 5B: 40% Scandium-doped aluminum nitride, Al_{0.6}Sc_{0.4}N providing a displacement field of 1.9 nm and a pressure field of +-465 kPa at a frequency of 3.492 MHz. Fig. 5C: A lead-zirconate-titanate (PZT) transducer providing a displacement field of 6 nm and a pressure field of +-1430 kPa at a frequency of 3.487 MHz.

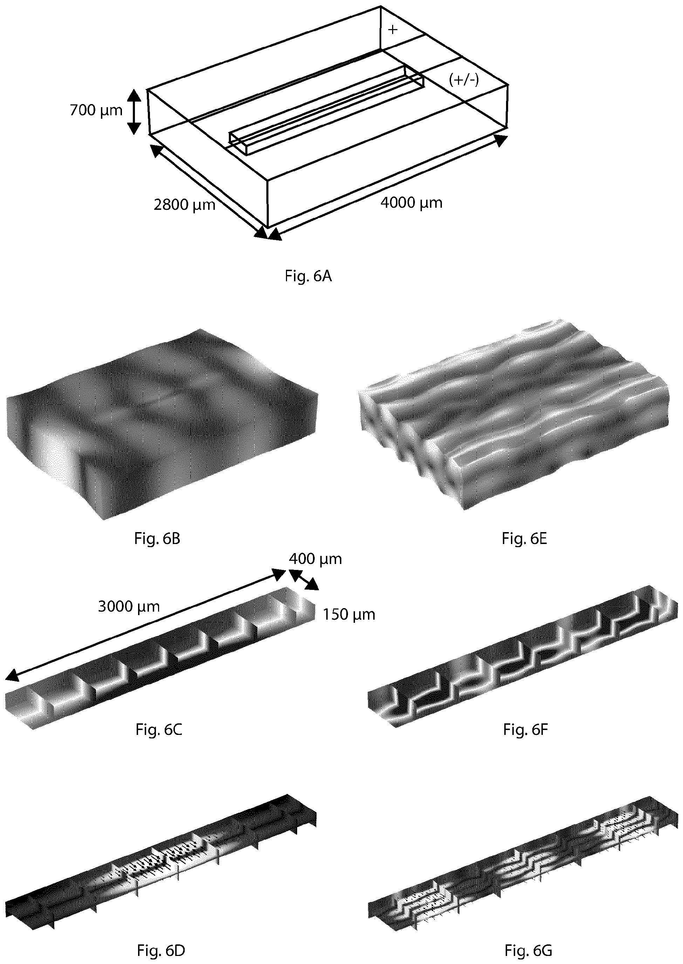

Fig. 6A-G show numerical simulations in 3D. Fig. 6A thus shows a 0.7 × 2.8 × 4 mm³ Pyrex substrate containing a 0.15 × 0.4 × 3 mm³ water-filled microchannel. The two thin film ultrasound transducers used are formed as a 2-µm Al N thin-film transducer actuated at 1 Vpp with a split top electrode placed on the top of the substrate and marked "+" and "(+/-)". Figs 6B, 6C and 6D show the displacement field (1.7 nm), the pressure field (+-300 kPa), and the radiation force (2.5 pN), respectively for unsymmetric (i.e., antiphase) actuation at 1.76 MHz. Figs. 6E, 6F, and 6G show the displacement field (0.8 nm), the pressure field (+-85 kPa), and the radiation force (0.4 pN), respectively for symmetric (i.e., in-phase) actuation at 3.83 MHz using a full top electrode. The 3D fields are similar to the ones calculated in the 2D cross section, but they have additional variations in the axial direction.

It should be noted that the above discussed simulations were made at a 1 Vpp actuation of the thin film ultrasound transducers. The acoustical energy in the device generally scales as the voltage squared. A higher voltage, such as 10 Vpp or 20 Vpp, or higher, will provide significantly stronger acoustofluidic effects.

### EXAMPLE 2 - Experimental and simulated actuation of glass acoustofluidic device using an Al N thin-film ultrasound transducer.

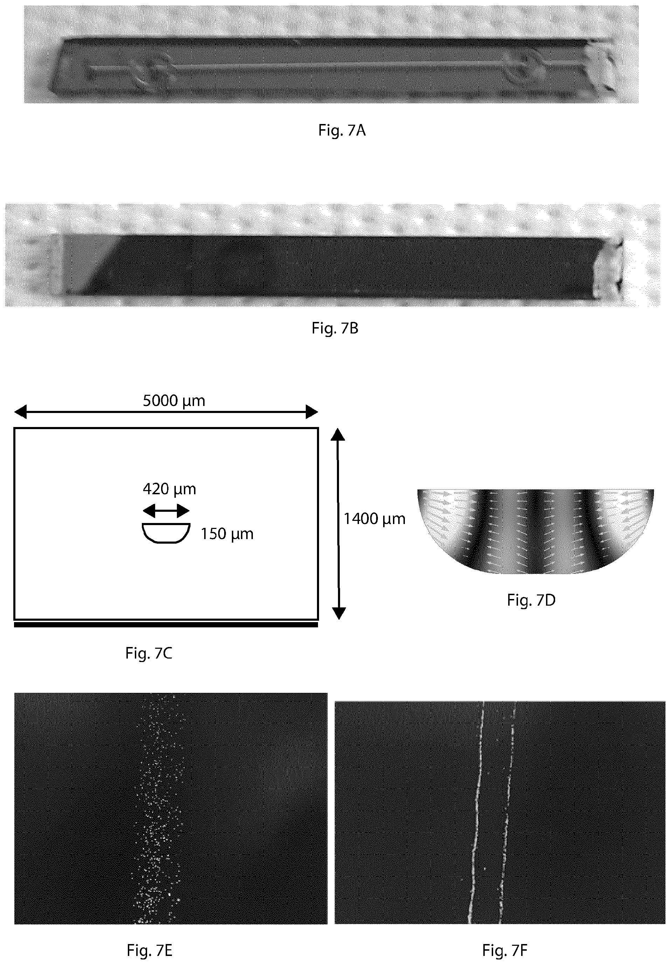

Fig. 7A and 7B show the front side and back side respectively of a 45 mm long glass substrate comprising a microfluidic cavity in the form of a channel and having the cross-sectional dimensions shown in Fig. 7C. The back side of the substrate is covered with a 1 µm Al N thin film ultrasound transducer with a ground electrode layer in contact with the backside of the substrate and full top electrode. Fig. 7D shows the numerical simulation of the substrate showing two vertical pressure nodes in with a radiation force of 1 pN at a resonance frequency of 3.60 MHz. Fig. 7E is a microscope photography of 5 µm diameter fluorescent polystyrene beads passing through the channel when the thin film ultrasound transducer is turned off. As seen, the beads are not ordered. Turning on the thin film ultrasound transducer found a resonance at 3.73 MHz, in which the beads are focused into two lines, see Fig. 7F, as predicted by the two pressure nodes shown in fig. 7D.

### EXAMPLE 3 - An external tunable inductance can increase power of the acoustofluidic device at a given resonance frequency for a given driving voltage.

A thin film ultrasound transducer, due to its construction, behaves almost purely as a capacitor. This is different from the conventional bulk ultrasound transducer which primarily behaves as an LCR circuit when at resonance. Further, as the resonances are substantially only in substrate, the resonances do not normally approach a phase of zero where the power delivery is at maximum. The system therefore has a large impedance around -90 degrees with only a few degrees in dips from small resonances towards zero phase. The power in the acoustofluidic device can be calculated as P = V_0^2*cos(Z_Phase)/Z_Abs, where P is the power, V_0 is the driving voltage, Z_Abs and Z_Phase is respectively the impedance absolute value and the impedance phase. The power is thus at maximum when the impedance phase and absolute value approaches zero.

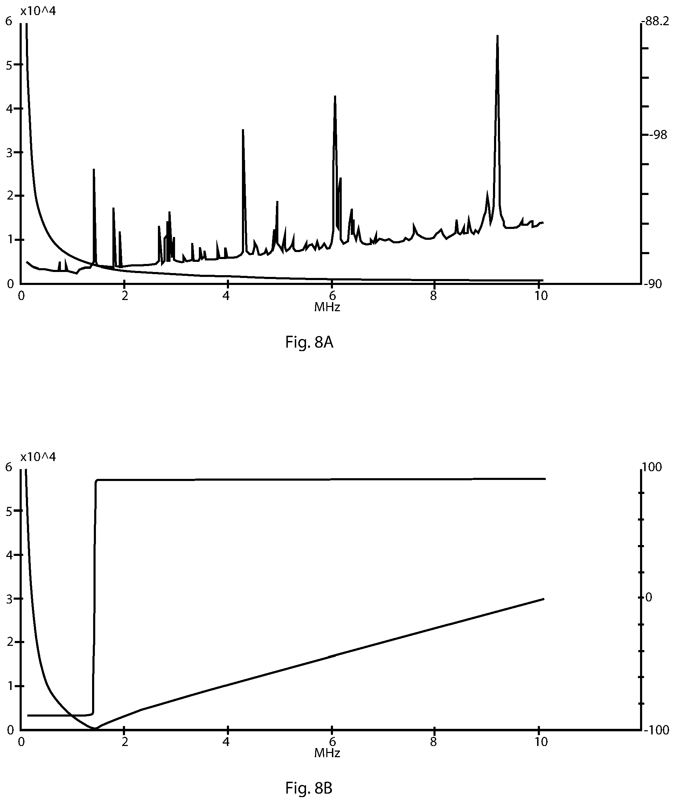

Fig. 8A shows the impedance spectrum of a 570 µm x 2000 µm x 2500 µm glass substrate with a 1 µm Al N thin film ultrasound transducer with frequency on the x axis and absolute value of the impedance on the left y axis and phase in degrees on the right y axis, the peaks corresponding to the right axis and the smooth curve corresponding to the left axis. As seen in the graph, the resonances, seen as peaks, do not reach up to a phase of zero, where maximum power output would be obtained. However, by adding an inductor in series the power output at a specific frequency can be amplified, by changing the behavior of the circuit into an RLC resonator (Resistor, Inductor, Capacitor) at that frequency.

In this example, in order to enhance the resonance at 1.4 MHz, a 3 mH inductor was added in series with the thin film ultrasound transducer. Fig. 8B shows the resulting impedance spectrum which now has a strong resonance at 1.4 MHz seen by the fact that the phase is close to zero and an appropriate dip in the absolute value of the impedance. Calculating the power for the resonance at 1.4 MHz for the two different setups shows a 130 times higher power when the inductor is used.

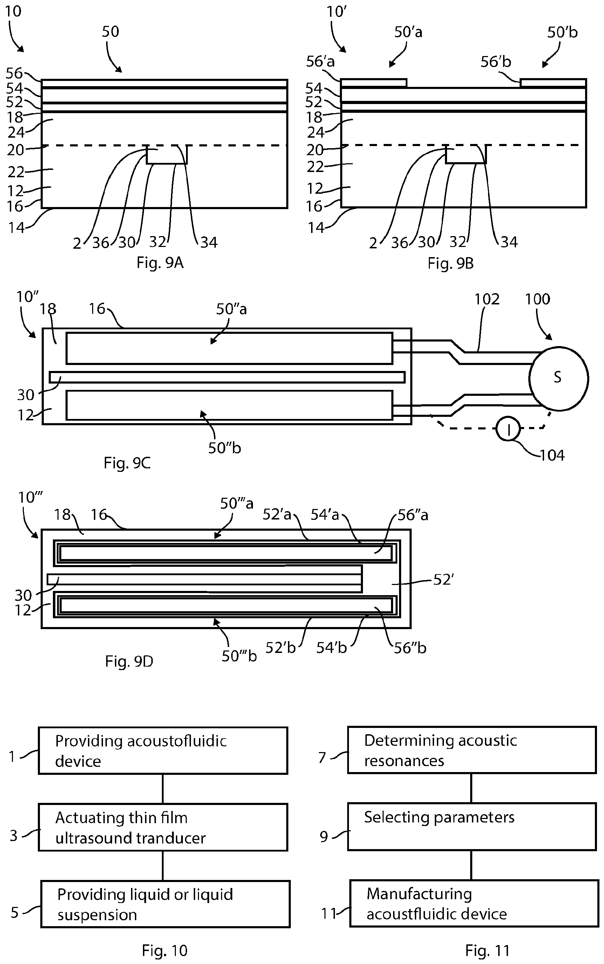

Fig. 9A schematically and in cross section shows the general construction of an acoustofluidic device 10 according to the first aspect of the technology proposed herein. Acoustofluidic device 10 comprises a substrate 12 having a bottom surface 14, first and second opposing side surfaces, one of which is designated 16, as well as a top surface 18, Optionally, as shown by the hatched line 20, the substrate 12 may be formed of base portion 22 and a lid portion 24. A microfluidic cavity in the form of a microfluidic channel 30 is provided in the substrate 12, the channel 30 having a bottom wall 32 and a top wall 34, as well as opposing first and second sidewalls, one of which is designated 36. For ease of manufacturing, as shown in Fig. 8A and 8B, the microfluidic channel is formed, for example by etching, into the base portion 22, whereby the lid portion 24 closes the microfluidic channel 30 to define the top wall 34 of the microfluidic channel 30. On the top surface 18 of the substrate 12 a thin film ultrasound transducer 50 is attached. Thin film ultrasound transducer 50 comprises a first (bottom) electrode layer 52 in acoustic contact with the top surface 18 of the substrate 12, a piezoelectric or electrostrictive layer 54 in contact with the first electrode layer 52, and a second (top) electrode layer 56. The thin film ultrasound transducer 50 is actuated, i.e., driven, by a drive circuit (one example being shown in FIG. 8C) so as to vibrate and cause the substrate 12 to vibrate at a resonance frequency of the substrate and the microfluidic channel 30 filled with a liquid or liquid suspension 2. Vibration of the substrate 12 at a resonance frequency gives rise to a pressure field in the channel 30 which can be used to perform acoustofluidic operations on the liquid or liquid suspension 2 positioned in, or caused to flow through, the microfluidic channel 30.

Fig. 9B schematically and in cross section shows the general construction of a second embodiment of an acoustofluidic device 10' according to the first aspect of the technology proposed herein. The acoustofluidic device 10' differs from the acoustofluidic device 10 in Fig. 8A in that the second (top) electrode layer 56 has been split into first and second portions 56'a and 56'b which portions, together with the first electrode layer 52 and the piezoelectric or electrostrictive layer 54, define two thin film ultrasound transducers 50a and 50'b which can be actuated symmetrically, i.e., in phase, or asymmetrically, i.e., out of phase such as in antiphase. Preferably, as shown in Fig. 8B, the first and second portions 56'a and 56'b only cover a fraction, such as about 25% or less each, of the piezoelectric or electrostrictive layer 54 in order to obtain a stronger displacement field and stronger acoustofluidic effects in the microfluidic channel 30.

Fig. 9C schematically shows a top view of an acoustofluidic device 10" similar to that in Fig 8B but differing in that separate first and second thin film ultrasound transducers 50"a and 50"b are provided spaced apart on the top surface 18 along the first and second side surfaces 16 so that the microfluidic channel 30 can be observed, imaged an optically interrogated through the top surface 18 of the substrate 12. A liquid or liquid suspension can be introduced into the microfluidic channel 30 through an inlet at one end (not shown) and removed from the microfluidic channel 30 through an outlet (not shown) at the other end. Also shown in Fig. 9C is a drive circuit 100 configured for actuating the first and second thin film ultrasound transducers 50"a and 50"b symmetrically (in-phase) or asymmetrically (out of phase such as in antiphase), the drive circuit 100 being electrically connected to the thin film ultrasound transducers 50"a and 50"b via electrical leads, one of which is designated 102, and which connect to the first and second electrode layers (not shown).

As discussed in example 3 and Figs. 8A-8B, it is advantageous to include an optional tunable inductor 104 in series in the connection with one or each of the thin film ultrasound transducer (only one shown in Fig. 9C). This is due to the primarily capacitive behavior of the thin film ultrasound transducers. By including a tunable inductor, more ultrasound energy can be delivered to the substrate from the thin film ultrasound transducer.

Fig. 9D schematically shows a top view of an acoustofluidic device 10''' similar to that in Fig. 8C but differing in that first and second thin film ultrasound transducers 50'''a and 50'''b have a common first bottom electrode layer 52' being generally U-shaped so as to form first and second parallel, partially interconnected, portions 52'a and 52'b, on which first and second layers of piezoelectric or electrostrictive layers 54'a and 54'b are provided, followed by first and second (top) electrode layers 56"a and 56"b.

Fig. 10 shows an embodiment of the method according to the second aspect of the technology proposed herein, including the steps of:
providing, designated the reference numeral 1, an acoustofluidic device comprising a substrate in which a microfluidic cavity is positioned and a thin film ultrasound transducer provided in acoustic contact with at least a part of one surface of the substrate for transferring ultrasonic vibrations to the substrate and causing the substrate to vibrate, actuating, designated the reference numeral 3, the at least one thin film ultrasound transducer at a frequency f that corresponds to an acoustic resonance peak of the substrate including the microfluidic cavity filled with a liquid suspension, and providing, designated the reference numeral 5, the liquid suspension in the microfluidic cavity to perform the acoustofluidic operation on the liquid suspension.

Fig. 11 shows an embodiment of the method according to the third aspect of the technology proposed herein, including the steps of: determining, designated the reference numeral 7, by calculation or simulation, the acoustic resonances of the substrate for each of a plurality of different combinations of parameter values of substrate parameters, the substrate parameters including substrate material, substrate dimensions, microfluidic cavity dimensions, microfluidic cavity positions within the substrate, properties of a liquid in the microfluidic cavity, positions for at least one thin film ultrasound transducer, and actuation frequency f, and selecting, designated the reference numeral 9, among the plurality of different combinations of the parameter values of the substrate parameters, a substrate material M, a set of substrate dimensions DS, a set of microfluidic cavity dimensions DC, a microfluidic cavity position PC within the substrate, properties of the liquid L in the microfluidic cavity, a position PU for at least one thin film ultrasound transducer, and an actuation frequency f, which yield acoustic resonance within the substrate including the microfluidic cavity, and manufacturing, designated the reference numeral 11, the acoustofluidic device made out of the substrate material M having the substrate dimensions DS and being provided with a microfluidic cavity having the microfluidic flow cavity DC and the microfluidic cavity position PC within the substrate.

### Feasible modifications of the technology proposed herein

The technology proposed herein is not limited to the embodiments described above and shown in the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims.

For instance, it shall be pointed out that structural aspects of embodiments of the acoustofluidic device according to the first aspect of the technology proposed herein shall be considered to be applicable to embodiments of the method according to the second aspect of the technology proposed herein, and vice versa.

It shall also be pointed out that all information about/concerning terms such as above, under, upper, lower, etc., shall be interpreted/read having the equipment oriented according to the figures, having the drawings oriented such that the references can be properly read. Thus, such terms only indicate mutual relations in the shown embodiments, which relations may be changed if the inventive equipment is provided with another structure/design.

It shall also be pointed out that even thus it is not explicitly stated that features from a specific embodiment may be combined with features from another embodiment, the combination shall be considered obvious, if the combination is possible.

Throughout this specification and the claims which follows, unless the context requires otherwise, the word "comprise", and variations such as "comprises" or "comprising", will be understood to imply the inclusion of a stated integer or steps or group of integers or steps but not the exclusion of any other integer or step or group of integers or steps.

## Claims

1. An acoustofluidic device (10) comprising:
- a substrate (12) in which a microfluidic cavity (30) is positioned, and
- a thin film ultrasound transducer (50) provided in acoustic contact with at least a part of one surface (18) of the substrate for transferring ultrasonic vibrations to the substrate and causing the substrate to vibrate, wherein the thin film ultrasound transducer preferably has a thickness of less than 100 µm, such as less than 50 µm, such as less than 5 µm, such as less than 2 µm.

2. The acoustofluidic device (10) according to claim 1, wherein the thickness of the thin film ultrasound transducer (50) is less than 10 µm, preferably 5 µm or less, such as 0.01 to 5 µm, more preferably 3 µm or less, such as 0.5 to 3 µm, more preferably 2 µm or less, such as 0.5 to 2 µm, such as 0.5 to 1.5 µm, such as 1 µm, or less than 1 µm.

3. The acoustofluidic device (10) according to claim 1 or 2, wherein the thin film ultrasound transducer comprises a layer of piezoelectric or electrostrictive material (54) placed between a first electrode layer (52) defining a first side of the thin film ultrasound transducer and a second electrode layer (56) defining a second side of the thin film ultrasound transducer, and wherein the first side of the thin film ultrasound transducer is provided in acoustic contact with the at least one part of the one surface (18) of the substrate (12).

4. The acoustofluidic device (10) according to any preceding claim, wherein the acoustofluidic device further comprises:
- a further thin film ultrasound transducer (50"b) provided in acoustic contact with at least a part of the one surface (18) of the substrate (12) for transferring ultrasonic vibrations to the substrate and causing the substrate to vibrate.

5. The acoustofluidic device (10) according to claim 3, wherein at least one of the first and second electrode layers is divided into at least two portions, thereby defining two separate electrodes (56'a, 56'b), which two electrodes, together with the layer of piezoelectric or electrostrictive material (54) and the electrode defined by the other of the first and second electrode layers, define the thin film ultrasound transducer (50'a) and a further thin film ultrasound transducer (50'b) as having a common layer of piezoelectric or electrostrictive material and one common electrode layer (52).

6. The acoustofluidic device (10) according to any of the claims 4-5, wherein the positions and extensions of the thin film ultrasound transducer (50"a) and the further thin film ultrasound transducer (50"b) are configured so as to allow the microfluidic cavity (30) to be observed or analyzed through the one surface (18),
and wherein preferably the thin film ultrasound transducer and the further thin film ultrasound transducer are positioned spaced apart at or along opposite borders of the one surface so as to allow the microfluidic cavity to be observed or analyzed through the part of the one surface between the thin film ultrasound transducer and the further thin film ultrasound transducer.

7. The acoustofluidic device (10) according to any preceding claim, wherein the thin film ultrasound transducer (50) comprises a piezoelectric or electrostrictive material selected from the group consisting of Zinc oxide, aluminum nitride, scandium-doped aluminum nitride, cerium oxides, and lead-zirconate-titanate.

8. The acoustofluidic device (10) according to any preceding claim, further comprising:
- a drive circuit (100) configured to actuate the thin film ultrasound transducer (50) at a frequency f that corresponds to an acoustic resonance peak of the substrate (12) including the microfluidic cavity (30) filled with a liquid or liquid suspension (2), and wherein the drive circuit preferably further comprises an inductor (104) connected in series with the thin film ultrasound transducer.

9. The acoustofluidic device (10) according to claim 8, wherein the acoustic resonance peak corresponds to three-dimensional volume resonance in the substrate (12) including the microfluidic cavity (30), which three-dimensional volume resonance cannot be described as a one- or two-dimensional resonance in the substrate, and wherein preferably the frequency f does not correspond to a resonance frequency of the microfluidic cavity alone.

10. The acoustofluidic device (10) according to any of the claims 8-9, wherein the acoustofluidic device comprises the further thin film ultrasound transducer (50'b, 50"b), and wherein the drive circuit (100) is further configured to actuate the thin film ultrasound transducer (50'a, 50"a) and the further thin film ultrasound transducer (50'b, 50"b) at the frequency f in phase, or preferably out of phase such as in antiphase, to each other.

11. A method of performing an acoustofluidic operation, comprising the steps of:
a. providing (1) an acoustofluidic device (10) comprising:
- a substrate (12) in which a microfluidic cavity (30) is positioned, and
- a thin film ultrasound transducer (50) provided in acoustic contact with at least a part of one surface (18) of the substrate for transferring ultrasonic vibrations to the substrate and causing the substrate to vibrate, wherein the acoustofluidic device preferably is an acoustofluidic device according to any of the preceding claims,
b. actuating (3) the thin film ultrasound transducer at a frequency f that corresponds to an acoustic resonance peak of the substrate including the microfluidic cavity filled with a liquid or a liquid suspension (2), and
c. providing (5) the liquid or liquid suspension in the microfluidic cavity to perform the acoustofluidic operation on the liquid or the liquid suspension.

12. The method according to claim 11, wherein
- step a comprises providing an acoustofluidic device (10) according to any of the claims 4-10, and
- step b comprises actuating the thin film ultrasound transducer (50'a) and the further thin film ultrasound transducer (50'b) at the frequency f in phase, or preferably out of phase such as in antiphase, to each other.

13. The method according to any of the claims 11-12, wherein the acoustofluidic operation is an acoustophoretic operation comprising focusing cells or other particles, suspended in a liquid suspension (2) within the microfluidic cavity (30), towards one or more discrete areas of the microfluidic cavity.

14. A method of producing an acoustofluidic device for performing an acoustofluidic operation, the acoustofluidic device comprising a substrate in which a microfluidic cavity is provided, comprising the steps of:
a. determining (7), experimentally, or by calculation or simulation, the acoustic resonances of the substrate for each of a plurality of different combinations of parameter values of substrate parameters, the substrate parameters including substrate material, substrate dimensions, microfluidic cavity dimensions, microfluidic cavity position within the substrate, properties of a liquid in the microfluidic cavity, position for at least one, preferably two, thin film ultrasound transducer in acoustic contact with at least a part of one surface of the substrate, and actuation frequency f, and
b. selecting (9), among the plurality of different combinations of the parameter values of the substrate parameters, a substrate material M, a set of substrate dimensions D_{S}, a set of microfluidic cavity dimensions D_{C}, a microfluidic cavity position P_{C} within the substrate, properties of the liquid L in the microfluidic cavity, a position P_{U} for the at least one, preferable two, thin film ultrasound transducer, and an actuation frequency f, which yield acoustic resonance within the substrate including the microfluidic cavity for performing the acoustofluidic operation, and
c. manufacturing (11) the acoustofluidic device made out of the substrate material M having the substrate dimensions D_{S} and being provided with a microfluidic cavity having the microfluidic cavity dimensions D_{C} and the microfluidic cavity position P_{C} within the substrate.

15. The method according to claim 14, wherein
- step a further comprises determining the acoustic force on a target particle throughout the substrate for each of the plurality of different combinations of parameter values of substrate parameters, and
- step b further comprises determining the set of microfluidic cavity dimensions D_{C} and the microfluidic cavity position P_{C} within the substrate so that the microfluidic cavity at least partly delimits a region of the substrate in which the acoustic force on the target particle is suitable for performing the acoustofluidic operation.
